# EUROPÄISCHE PATENTSCHRIFT

(11) **EP 1 294 741 B1**
(45) Veröffentlichungstag und Bekanntmachung des Hinweises auf die Patenterteilung: **16.02.2005**
(21) Anmeldenummer: 01960319.0
(22) Anmeldetag: 15.06.2001
(51) Int. Cl.: C07K 5/06, A61K 38/04, C07C 257/00

(54) **DIPEPTIDISCHE HEMMSTOFFE FÜR DEN GERINNUNGSFAKTOR XA**
DIPEPTIDE INHIBITORS FOR THE BLOOD-CLOTTING FACTOR XA
INHIBITEURS DIPEPTIDE DU FACTEUR DE COAGULATION XA

(30) Priorität: 15.06.2000 DE 10029015
(43) Veröffentlichungstag der Anmeldung: 26.03.2003
(73) Patentinhaber: Curacyte AG, 80339 München (DE)
(72) Erfinder: STÜRZEBECHER, Jörg, D-99094 Erfurt (DE); STEINMETZER, Torsten, D-07743 Jena (DE); KÜNZEL, Sebastian, D-69126 Heidelberg (DE); SCHWEINITZ, Andrea, D-07745 Jena (DE)
(74) Vertreter: Bösl, Raphael, Dr. rer. nat., Dipl.-Chem.
(86) Internationale Anmeldenummer: PCT/EP2001/006814
(87) Internationale Veröffentlichungsnummer: WO 2001/096366

(56) Entgegenhaltungen:
- WO-A-00/58346
- WO-A-94/29336
- WO-A-95/29189
- WO-A-96/25426
- US-A- 5 705 487
- US-A- 5 707 966
- US-A- 5 710 130
- US-A- 5 726 159
- US-A- 5 914 319

## Beschreibung

Die Erfindung betrifft neue Hemmstoffe für den Gerinnungsfaktor Xa zur Behandlung von kardiovaskulären Erkrankungen und zur Verhinderung thromboembolischer Ereignisse.

Die gegenwärtig klinisch eingesetzten Antikoagulantien vom Heparin-Typ bzw. die Vitamin-K-Antagonisten werden nicht allen Anforderungen an ein "ideales" Antithrombotikum gerecht. Deshalb wird mit kleinmolekularen Hemmstoffen der Gerinnungsenzyme, speziell von Thrombin und Faktor Xa (F Xa), nach Alternativen gesucht. Ein besonderer Vorteil von F Xa-Hemmstoffen im Vergleich zu Thrombin-Hemmstoffen könnte die geringere Blutungsneigung sein, die sich bei verschiedenen Tierversuchen gezeigt hat. So wurde bei antithrombotisch effektiven Dosen die Blutungszeit nur minimal beeinflußt (J.M. Herbert et al., J. Pharmacol. Exp. Ther. 276, 1030-1038, 1996; K. Sato et al., Br. J. Pharmacol. 123, 92-96, 1998).

Die ersten nichtpeptidischen Verbindungen mit hoher Affinität für F Xa waren symmetrische Bis-benzamidine (Kᵢ = 13 nM für die wirksamste Verbindung BABCH) (J. Stürzebecher et al., Thromb. Res. 54, 245-252, 1998). Auch das Naphthamidin-Derivat DX-9065a besitzt zwei basische Gruppen und hemmt F Xa selektiv mit einem Kᵢ = 24 nM (T. Hara et al., Thromb. Haemost. 71, 314-319, 1994). Der mit DX-9065a strukturell verwandte Inhibitor YM-60828 (K. Sato et al. Eur. J. Pharmacol. 339, 141-146, 1997) ist noch wirksamer (Kᵢ = 1.3 nM). Inzwischen wurde eine ganze Reihe weiterer bis-basischer Verbindungen beschrieben, bei denen z. B. zwei Benzamidin-Reste über einen Oxazolin-Ring (Kᵢ = 18 nM) (M.L. Quan et al., Bioorg. Med. Chem. Lett. 7, 2813-2818, 1997) bzw. eine Carboxymethylalkyl-Kette (Kᵢ = 34 nM) verknüpft sind (T.P. Maduskuie et al., J. Med. Chem. 41, 53-62, 1998). Nachteil der bis-basischen Verbindungen ist insbesondere die geringe Bioverfügbarkeit nach oraler Gabe.

Auch Hemmstoffen für F Xa, die nur eine basische Gruppe enthalten, wurden beschrieben. N-substituierte Amidino-phenoxypyridine (Kᵢ = 0,11 nM für BX-807834) wurden auf der Basis von BABCH entwickelt (R. Mohan et al., Bioorg. Med. Chem. Lett. 8, 1877-1882, 1998; G.B. Phillips et al. J. Med. Chem. 41, 3557-3562, 1998). Amide des Nα-Adamantyloxycarbonyl-3-amidinophenylalanins (Kᵢ = 74 nM für die wirksamste Verbindung) sind selektive Hemmstoffe des F Xa (S. Sperl et al., Biol. Chem. 381, 321-329, 2000), während Nα-arylsulfonyl-aminoacylierte Ester des 3-Amidinophenylalanins eine geringe Hemmwirkung (Kᵢ = 840 nM für TAPAM) besitzen (J. Stürzebecher et al., Thromb. Res. 54, 245-252, 1998). Die WO 96/10022 offenbart Hemmstoffe, die überhaupt keine starke Ladung mehr besitzen (Kᵢ = 3,0 nM für die wirksamste Verbindung).

Bisher wurden nur wenige Peptide als Hemmstoffe für F Xa beschrieben, die sich von der Substrat-Sequenz Ile-Glu-Gly-Arg ableiten. Die von Kettner und Shaw (Thromb. Res. 22, 645-652, 1981) beschriebenen Chlormethylketone hemmen F Xa irreversibel und sind nicht für in vivo-Anwendungen geeignet. Dagegen sind die Peptide SEL 2489 (Kᵢ = 25 nM) und SEL 2711 (Kᵢ = 3 nM) außerordentlich wirksam (J. A. Ostrem et al., Biochemistry 37, 1053-1059, 1998). Auch einige Peptidyl-Arginin-Aldehyde wurden beschrieben, die neben Argininal in P1-Position ein D-Arginin bzw. eine unnatürliche basische Aminosäure in P3 besitzen (Z. H. Jonathan, Bioorg. Med. Lett. 9, 3459-3464, 1999.) Dagegen sind bisher keine Peptidyl-Agmatin-Derivate als Hemmstoffe für F Xa bekannt, obwohl dieser Inhibitor-Typ bei der Weiterentwicklung von Thrombin-Inhibitoren zu erheblichen Fortschritten geführt hat. Dabei waren die Erfolge bei Verbindungen des D-Phe-Pro-Arg-Typs mit C-terminalem Agmatin und davon abgeleiteten Derivaten besonders bemerkenswert. Es wurden picomolare Kᵢ-Werte für die Thrombin-Hemmung erreicht und die orale Bioverfügbarkeit verbessert (T.J. Tucker et al., J. Med. Chem. 40, 1565-1569 und 3687-3693, 1997). Dabei wurde allerdings keine Hemmung des F Xa beobachtet. So hemmt Melagatran, welches C-terminal einen 4-Amidino-benzylamin-Rest besitzt und sehr unspezifisch ist, F Xa mit einem Kᵢ = 2,8 µM. Dagegen werden Trypsin (Kᵢ = 4,0 nM) und Thrombin (Kᵢ = 2,0 nM) mehr als drei Größenordnungen stärker gehemmt (D. Gustafsson et al., Blood Coagul. Fibrinolysis 7, 69-79, 1996).

Der Erfindung liegt die Aufgabe zu Grunde, einen auch für therapeutische Anwendungen geeigneten Wirkstoff anzugeben, der den Gerinnungsfaktor Xa mit hoher Aktivität und Spezifität hemmt und der mit möglichst geringem Syntheseaufwand herstellbar ist.

Überraschend wurde gefunden, dass acyliertes Amidino-benzylamin gemäß der in den Patentansprüchen 1 bis 7 angeführten Verbindungen der Formel I, insbesondere Verbindungen des 4-Amidino-benzylamins, bei denen X, R₁, R₂ und R₃ natürliche und/oder unnatürliche Aminosäuren ergeben, Faktor Xa sehr wirksam und selektiv inaktivieren und die Gerinnung von menschlichem Blutplasma effektiv hemmen. Einen besonders aktiven Hemmstoff von Faktor Xa bildet dabei Amidino-benzylamin, wenn die Amidinogruppe in 4-Position steht, als Aminosäuren Glycin und D-Serin-tert.-butylether gebunden sind und wenn die Verbindung eine N-terminale Schutzgruppe R₄ aus einem Aryl- bzw. Aralkyl-sufonyl-Rest aufweist. Neben Faktor Xa wurden durch die Glycin-Derivate andere Enzyme deutlich weniger gehemmt, so dass die erfindungsgemäßen Derivate des Amidinobenzylamins eine neue Gruppe von hochaktiven und sehr selektiven F Xa-Hemmstoffen darstellen. Im Gegensatz dazu hemmen Verbindungen, die als R₁ kein H tragen (z. B. Alanin-Deriate) Faktor Xa nicht mehr selektiv, sondern sind auch starke Hemmstoffe von Trypsin, Thrombin und Plasmin.

Die Verbindungen liegen in der Regel als Salze mit Mineralsäuren, bevorzugt als Hydrochloride, vor oder als Salze mit geeigneten organischen Säuren.

Des weiteren betreffen die US-Patente US 5,914,319, US 5,726,159, US 5,710,130, US 5,707,966 und US 5,705,487 spezifische Peptidderivate als Thrombininhibitoren, wobei bestimmte Verbindungen auch Faktor Xa inhibieren. In der WO 96/25426 werden spezifische dipeptidische Amidine als Thrombin-Inhibitoren und in der WO 95/29189 spezifische Peptidderivate als Faktor Xa Inhibitoren offenbart. Auch in der WO 94/29336 werden spezifische Inhibitoren von Trypsin-ähnlichen Serinproteasen und in der WO 00/58346 werden spezifische N-Sulfonyl-Dipeptid-Derivate vorgeschlagen.

Die Verbindungen der allgemeinen Formel I können in prinzipiell bekannter Weise, wie nachfolgend beschrieben, hergestellt werden:

Die Ausgangsverbindung 4-Cyanobenzylamin wird über Gabrielsynthese (G. Wagner und I. Wunderlich, Pharmazie 32, 76-77, 1977; B.C. Bookser und T.C. Bruice, J. Am. Chem. Soc. 113, 4208-4218, 1991) aus 4-Cyanobenzylbromid hergestellt Aus dem so hergestellten 4-Cyanobenzylamin wird das Boc-geschützte Acetyloxamidino-benzylamin gewonnen. Die Ankopplung der weiteren Aminosäuren und, der Schutzgruppe R₄ erfolgt mittels Standardkopplungsmethoden mit Boc als N-terminale Schutzgruppe. Die zweite Aminosäure kann auch direkt als N-aryl- bzw. N-aralkyl-sulfonyl-geschützte Aminosäure gekoppelt werden. Die Peptidanaloga werden sequentiell, beginnend vom Acetyloxamidino-benzylamin, aufgebaut. Die meisten Produkte kristallisierten gut und lassen sich damit einfach reinigen. Die Reinigung der Hemmstoffe erfolgt in der letzten Stufe über präparative, reversed-phase HPLC.

Des weiteren betrifft die Erfindung die in den Ansprüchen 8 und 9 bezeichneter Verwendung der erfindungsgemäßen Hemmstoffe.

Die Erfindung soll nachstehend anhand von drei Ausführungsbeispielen näher erläutert werden:

### Ausführungsbeispiel 1:

### Synthese von Benzylsulfonyl-D-Ser(Bz)-Gly-4-Amidino-benzylamid x HCl

### 1.1 Boc-4-Cyano-benzylamid

20 g (0,151 mol) 4-Cyano-benzylamin wurden in 300 ml H₂O, 150 ml Dioxan und 150ml 1 N NaOH gelöst. Unter Eiskühlung wurden 37,5 ml Di-tert.-butyldicarbonat zugetropft und eine Stunde bei 0 °C sowie weitere 24 Std. bei Raumtemperatur gerührt. Das Dioxan wurde im i.V. entfernt und der wässrige Rückstand 3-mal mit Essigester extrahiert. Die vereinigten Extrakte wurden 3-mal mit 5 %-iger KHSO₄- und 3-mal mit gesättigter NaCl-Lösung gewaschen, über Na₂SO₄ getrocknet und i.V. eingeengt (weiße Kristalle). HPLC: Acetonitril/H₂O, Elution bei 44,1 % Acetonitril; Ausbeute: 30,48 g (0,131 mol), 87 %.

### 1.2 Boc-4-Acetyloxamidino-benzylamid

Nach Judkins et al. (Synthetic Comm. 26, 4351-4367, 1996) wurden 30,48 g (0,131 mol) Boc-4-Cyano-benzylamid mit 13,65 g (0,197 mol) Hydroxylamin x HCl und 34 ml (0,197 mol) DIEA in 300 ml abs. Ethanol gelöst. Es wurde 2 Std. unter Rückfluss gekocht und über Nacht bei Raumtemperatur gerührt. Danach wurde der Ansatz i.V. eingeengt, der Rückstand in ca. 200 ml Essigsäure gelöst und mit 18,67 ml (0,197 mol) Essigsäureanhydrid versetzt. Nach 1 Std. wurde erneut eingeengt, in Essigester gelöst und bei 0 °C je 3-mal mit 5 %iger KHSO₄und gesättigter NaCl-Lösung gewaschen. Nach dem Trocknen über Na₂SO₄ und Einengen i.V. fiel ein weißes Pulver an. HPLC: Acetonitril/H₂O, Elution bei 32,0 % Acetonitril; Ausbeute: 31,3 g (0,102 mol) 78 %.

### 1.3 4-Acetyloxamidino-benzylamin x HCl

5 mmol Boc-4-Acetyloxamidino-benzylamid werden in 20 ml 1 N HCl in Eisessig gelöst und 45 min bei Raumtemperatur stehen gelassen. Dann wird i.V. weitgehend eingeengt, das Produkt mit trockenem Diethylether gefällt, abgefrittet, nochmals mit frischem Ether gewaschen und i.V. getrocknet. Auf Grund der quantitativen Umsetzung wurde das Produkt ohne weitere Reinigung für den nächsten Syntheseschritt eingesetzt.

### 1.4 Boc-Gly-4-Acetyloxamidino-benzylamid

Die Kopplung von Boc-Gly-OH (Orpegen, Heidelberg) an 4-Acetyloxamidino-benzylamin erfolgte nach Frérot et al. (Tetrahedron 47, 259 ff., 1991). Dazu wurden 2,064 g (9,3 mmol) 4-Acetyloxamidino-benzylamin x HCl und 1,629 g (9,3 mmol) Boc-Gly-OH in ca. 25 ml DMF gelöst. Bei 0 °C wurden dann 4,84 g (9,3 mmol) PyBOP und 3,878 ml (27,9 mmol) TEA zugegeben und der pH-Wert mit TEA auf 9 eingestellt. Nach 1 Std. Rühren bei Raumtemperatur wurde i.V. eingeengt, in Essigester aufgenommen und je 3-mal sauer, basisch und neutral gewaschen, getrocknet und eingeengt. Ausbeute: 3 g (8,2 mmol) 88 %.

### 1.5 Boc-Gly-4-Amidino-benzylamid x AcOH

3 g (8,2 mmol) Boc-Gly-4-Acetyloxamidino-benzylamid wurden in 200 ml 90 %iger Essigsäure gelöst. Anschließend wurden unter Argon 300 mg 10 % Palladium auf Aktivkohle zugesetzt. Argon wurde durch eine Wasserstoffatmosphäre ersetzt und der Ansatz unter kräftigem Rühren 24 Std. hydriert. Der Katalysator wurde abfiltriert und das Filtrat i.V. eingeengt. Ausbeute: 2,9 g (7,9 mmol) 96 %.

### 1.6 H-Gly-4-Amidino-benzylamid x 2 HCl

2,9 g (7,9 mmol) Boc-Gly-4-Amidino-benzylamid wurden in 100 ml 1 N HCl in Eisessig gelöst und 45 min bei Raumtemperatur stehen gelassen. Dann wurde i.V. weitgehend eingeengt und mit trockenem Diethylether gefällt, danach abgefrittet und das Produkt nochmals mit frischem Ether gewaschen. Nach Trocknen des Produkts i.V. wurde es ohne weitere Aufreinigung für die Synthese nach Punkt 1.8 eingesetzt.

### 1.7 Benzylsulfonyl-D-Ser(Bz)-OH

229 mg (1,173 mmol) H-D-Ser(Bz)-OH und 408 µl (2,345 mmol) DIEA wurden in 50 ml 50 % Acetonitril gelöst. Dann wurden 335 mg (1,76 mmol) Benzylsulfonylchlorid zugegeben und 12 Std. bei Raumtemperatur gerührt. Es wurde i.V. eingeengt, mit Essigester aufgenommen und je 3-mal sauer und neutral gewaschen. Nach Trocknen über Natriumsulfat wurde i.V. eingeengt. Ausbeute: 289 mg (0,827 mmol) 71 %.

### 1.8 Benzylsulfonyl-D-Ser(Bz)-Gly-4-Amidino-benzylamid x TFA

151 mg (0,433 mmol) Benzylsulfonyl-D-Ser(Bz)-OH und 121 mg (0,433 mmol) H-Gly-4-Amidino-benzylamid x 2 HCl wurden in wenig abs. DMF gelöst. Unter Eiskühlung wurden 225 mg (0,433 mmol) PyBOP und 230 µl (1,32 mmol) DIEA zugegeben. Nach 1 Std. Rühren bei Raumtemperatur wurde i.V. eingeengt und das Produkt über HPLC gereinigt (Acetonitril/H₂O, 0,1% Trifluoressigsäure, Elution bei 37,4 % Acetonitril). Ausbeute: 232 mg (0,356 mmol) 82 %.

### Ausführungsbeispiel 2:

### Hemmung von F Xa durch ausgewählte Verbindungen mit Y = Amidino

| R₄ | Konfiguration R₃ | R₃ | R₂ | X-R₁ | Position Amidino | Kᵢ, µM |
|---|---|---|---|---|---|---|
| H | L | CH₂-OH | H | CH₂ | 4 | > 1000 |
| Boc | L | CH₂-OH | H | CH₂ | 4 | 110 |
| H | D | CH₂-OH | H | CH₂ | 4 | >1000 |
| Ac | D | CH₂-OH | H | CH₂ | 4 | >1000 |
| Bz-SO₂ | D | CH₂-OH | H | CH₂ | 4 | 3,0 |
| Bz-SO₂ | D | CH₂-O-Bz | H | CH₂ | 4 | 0,050 |
| Bz-SO₂ | D | CH₂-O-tBu | H | CH₂ | 4 | 0,030 |
| Bz-SO₂ | D | CH₂-O-tBu | H | CH₂-CH₃ | 4 | 0,044 |
| H | D | CH₂-O-Bz | H | CH₂ | 3 | 140 |
| Boc | D | CH₂-O-Bz | H | CH₂ | 3 | 93 |
| Bz-SO₂ | D | CH₂-O-Bz | H | CH₂ | 3 | 84 |

### Bestimmung der Hemmwirkung

Zur Bestimmung der Hemmwirkung wurden 200 µI Tris-Puffer (0,05 M, 0,154 M NaCI, 5% Ethanol, pH 8,0; enthält den Inhibitor), 25 µI Substrat (Moc-D-Nle-Gly-Arg-pNA in H₂O Pentapharm Ltd., Basel, Schweiz) und 50 µl F Xa (vom Rind, Diagnostic Reagents Ltd, Thame, GB) bei 25 °C inkubiert. Nach 3 min wurde die Reaktion durch Zugabe von 25 µI Essigsäure (50%) unterbrochen und die Absorption bei 405 nm mittels Microplate Reader (MR 5000, Dynatech, Denkendorf, Deutschland) bestimmt. Die Kᵢ-Werte wurden nach Dixon (Biochem. J. 55, 170-171, 1953) durch lineare Regression mittels eines Computerprogramms ermittelt. Die Kᵢ-Werte sind das Mittel aus mindestens drei Bestimmungen.

Ausführungsbeispiel 3:

### Hemmung der Gerinnung von humanem Plasma durch Benzylsulfonyl-D-Ser(Bz)-Gly-4-Amidino-benzylamid

| Konzentration µM | Verlängerung der Gerinnungszeit (%) | |
|---|---|---|
| | aPTT | PT |
| 3,3 | 385 | 386 |
| 1,7 | 260 | 266 |
| 0,83 | 185 | 198 |
| 0,42 | 146 | 153 |
| 0,21 | 122 | 127 |
| 0,1 | 111 | 119 |

### Bestimmung der Gerinnungshemmung

Zur Bestimmung der Prothrombin-Zeit (PT) wurden 100 µI Thromboplastin (Dade, Unterschleißheim) und 100 µl Inhibitor, gelöst in CaCl₂ (0,05 M, 5 % Ethanol) bei 37 °C für 2 min inkubiert und die Gerinnung durch Zugabe von 100 µl humanem Citrat-Plasma gestartet. Zur Bestimmung der aktivierten partiellen Thromboplastin-Zeit (aPTT) wurden 100 µl humanes Citrat-Plasma mit 100 µl aPTT-Reagenz (Roche Diagnostics, Mannheim) bei 37 °C für 3 min inkubiert und die Gerinnung durch Zugabe von 100 µl Inhibitor, gelöst in CaCl₂ (0,05 M, 5 % Ethanol) gestartet. Die Gerinnungszeiten wurden mit dem Koagulometer Thrombotrack (Immuno, Heidelberg) bestimmt.

### Verwendete Abkürzungen:

- Ac: Acetyl
- Boc: tert.-Butyloxycarbonyl
- Bz: Benzyl
- DIEA: Diisopropylethylamin
- DMF: N,N-Dimethylformamid
- i.V.: im Vakuum
- PyBOP: Benzotriazol-1-yl-N-oxy-tris(pyrrolidino) phosphoniumhexafluorophosphat

- TEA: Triethylamin
- TFA: Trifluoressigsäure
- THF: Tetrahydrofuran
- tBu: tert.-Butyl

## Patentansprüche

1. Hemmstoff für den Gerinnungsfaktor Xa der allgemeinen Formel I: 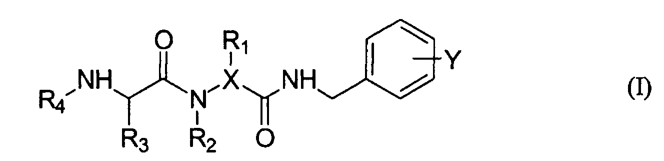 in denen der Substituent Y in 3- oder 4-Position vorkommt und eine Amidino-Gruppe 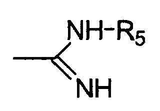 darstellt, in welcher R₅ ein H, ein OH oder ein Carbonylrest -CO-Rₐ bzw. Oxycarbonylrest -COO-Rₐ ist, wobei Rₐ ein verzweigtes oder unverzweigtes Alkyl mit 1-16 C-Atomen, ein substituierter oder unsubstituierter Aryl- oder Heteroarylrest oder ein substituierter oder unsubstituierter Aralkyl- oder Heteroaralkylrest ist,
X eine CH-Gruppe oder N ist,
R₁ als H oder als ein verzweigtes oder unverzweigtes Alkyl mit 1-8 C-Atomen oder als ein (CH₂)ₙ-OH mit n = 1-5 ausgebildet ist,
R₂ gleich H oder ein verzweigtes oder unverzweigtes Alkyl mit 1-8 C-Atomen ist,
R₃ gleich (CH₂)ₙ-O-R₆, (CH₂)ₙ-S-R₆ bzw. (CH₂)ₙ-NH-R₆ mit n = 1-5 ist und R₆ ein verzweigtes oder unverzweigtes Alkyl mit 1-16 C-Atomen, einen substituierten oder unsubstituierten Aryl- oder Heteroarylrest bzw. einen substituierten oder unsubstituierten Aralkyl- oder Heteroaralkylrest darstellt, und
R₄ einen Sulfonylrest -SO₂-R_{b} darstellt, wobei R_{b} ein verzweigtes oder unverzweigtes Alkyl mit 1-16 C-Atomen, ein substituierter oder unsubstituierter Aryl- oder Heteroarylrest, ein substituierter oder unsubstituierter Aralkyl- oder Heteroaralkylrest, ein Adamantyl-, ein Campher- oder ein Cyclohexylmethylrest ist.

2. Hemmstoff gemäß Anspruch 1, **dadurch gekennzeichnet, dass** der Substituent Y in 4-Position steht.

3. Hemmstoff gemäß Anspruch 1 oder 2, **dadurch gekennzeichnet, dass** X gleich CH und R₁ gleich H oder CH₂-OH ist.

4. Hemmstoff gemäß einem der Ansprüche 1 bis 3, **dadurch gekennzeichnet, dass** R₃ gleich CH₂-O-R₆ ist.

5. Hemmstoff gemäß Anspruch 4, **dadurch gekennzeichnet, dass** R₆ ein verzweigter Alkylrest oder ein unsubstituierter Aralkylrest ist.

6. Hemmstoff gemäß einem der Ansprüche 1 bis 5, **dadurch gekennzeichnet, dass** R₂ gleich H ist.

7. Hemmstoff gemäß einem der Ansprüche 1 bis 6, **dadurch gekennzeichnet, dass** R₄ ein Aralkylsulfonyl-Rest ist.

8. Verwendung eines Hemmstoffs gemäß einem der Ansprüche 1 bis 7 zur Herstellung von oral, subkutan, intravenös bzw. transdermal verabreichbaren Arzneimitteln zur Verhinderung bzw. Behandlung thromboembolischer Erkrankungen.

9. Verwendung gemäß Anspruch 8, **dadurch gekennzeichnet, dass** der Hemmstoff als Arzneimittel in Form von Tabletten, Dragées, Kapseln, Pellets, Suppositorien, Lösungen oder Pflaster etc. eingesetzt werden.

## Claims

1. Inhibitor for the clotting factor Xa of the general formula I: 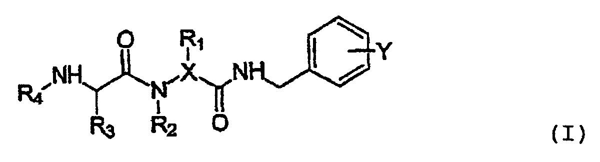 in which the substituent Y occurs in the 3- or 4-position and is an amidino group 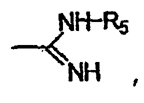 in which R₅ is an H, an OH or a carbonyl radical -CO-Rₐ or oxycarbonyl radical -COO-Rₐ, where Rₐ is a branched or unbranched alkyl having 1-16 C atoms, a substituted or unsubstituted aryl or heteroaryl radical or a substituted or unsubstituted aralkyl or heteroaralkyl radical,
X is a CH group or N,
R₁ is H or a branched or unbranched alkyl having 1-8 C atoms or (CH₂)ₙ-OH where n = 1-5,
R₂ is H or a branched or unbranched alkyl having 1-8 C atoms,
R₃ is (CH₂)ₙ-O-R₆, (CH₂)ₙ-S-R₆ or (CH₂)ₙ-NH-R₆ where n = 1-5 and R₆ is a branched or unbranched alkyl having 1-16 C atoms, a substituted or unsubstituted aryl or heteroaryl radical or a substituted or unsubstituted aralkyl or heteroaralkyl radical, and
R₄ is a sulfonyl radical -SO₂-R_{b}, where R_{b} is a branched or unbranched alkyl having 1-16 C atoms, a substituted or unsubstituted aryl or heteroaryl radical, a substituted or unsubstituted aralkyl or heteroaralkyl radical, an adamantyl radical, a camphor radical or a cyclohexylmethyl radical.

2. Inhibitor according to Claim 1, **characterized in that** the substituent Y is in the 4-position.

3. Inhibitor according to Claim 1 or 2, **characterized in that** X is CH and R₁ is H or CH₂-OH.

4. Inhibitor according to one of Claims 1 to 3, **characterized in that** R₃ is CH₂-O-R₆

5. Inhibitor according to Claim 4, **characterized in that** R₆ is a branched alkyl radical or an unsubstituted aralkyl radical.

6. Inhibitor according to one of Claims 1 to 5, **characterized in that** R₂ is H.

7. Inhibitor according to one of Claims 1 to 6, **characterized in that** R₄ is an aralkyl sulfonyl radical.

8. Use of an inhibitor according to one of Claims 1 to 7 for the production of orally, subcutaneously, intravenously or transdermally administrable medicaments for the prevention or treatment of thromboembolic diseases.

9. Use according to Claim 8, **characterized in that** the inhibitor is employed as medicament in the form of tablets, coated tablets, capsules, pellets, suppositories, solutions or patches, etc.

## Revendications

1. Inhibiteur de facteur de coagulation Xa de formule générale I: 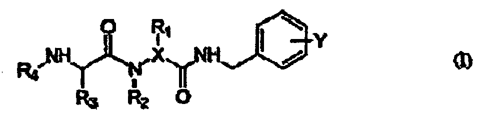 où le substituant Y se trouve en position 3 ou 4 et représente un groupe amidino 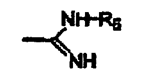 dans lequel R₅ est un H, OH ou un radical carbonyle -CO-R₈ ou bien un radical oxycarbonyle -COO-R₈, R₈ étant un alkyle ramifié ou non ramifié comportant 1 à 16 atomes de C, un radical aryle ou hétéroaryle substitué ou non substitué, ou un radical aralkyle ou hétéroaralkyle substitué ou non substitué,
X est un groupe CH ou N,
R₁ est formé de H ou d'un alkyle ramifié ou non ramifié avec 1 à 8 atomes de C ou d'un (CH₂)ₙ-OH avec n= 1 à 5,
R₂ est un H ou un radical alkyle ramifié ou non ramifié comportant 1 à 8 atomes de C,
R₃ est (CH₂)ₙ-O-R₆, (CH₂)ₙ-S-R₆ ou bien (CH₂)ₙ-NH-R₆ où n = 1 à 5, et R₆ représente un alkyle ramifié ou non ramifié avec 1 à 16 atomes de C, un radical aryle ou hétéroaryle substitué ou non substitué ou bien un radical aralkyle ou hétéroaralkyle substitué ou non substitué, et
R₄ représente un radical sulfonyle -SO₂-R_{b}, R_{b} étant un radical alkyle ramifié ou non ramifié comportant 1 à 16 atomes de C, un radical aryle ou hétéroaryle substitué ou non substitué, un radical aralkyle ou hétéroaralkyle substitué ou non substitué, un groupe adamantyle, camphre ou cyclohexylméthyle.

2. Inhibiteur selon la revendication 1, **caractérisé en ce que** le substituant Y se trouve en position 4.

3. Inhibiteur selon la revendication 1 ou 2, **caractérisé en ce que** X est CH et R₁ est H ou CH₂OH.

4. Inhibiteur selon l'une quelconque des revendications 1 à 3, **caractérisé en ce que** R₃ est CH₂-O-R₆.

5. Inhibiteur selon la revendication 4, **caractérisé en ce que** R₆ est un radical alkyle ramifié ou un radical aralkyle non substitué.

6. Inhibiteur selon l'une quelconque des revendications 1 à 5, **caractérisé en ce que** R₂ est un atome de H.

7. Inhibiteur selon l'une quelconque des revendications 1 à 6, **caractérisé en ce que** R₄ est un radical aralkylsulfonyle.

8. Utilisation d'un inhibiteur selon l'une quelconque des revendications 1 à 7, pour la fabrication de médicaments, pouvant être administrés par voie orale, sous-cutanée, intraveineuse ou transdermique, pour la prévention ou le traitement de maladies thromboemboliques.

9. Utilisation selon la revendication 8, **caractérisé en ce que** l'inhibiteur est utilisé comme médicament sous forme de comprimés, dragées, capsules, pastilles, suppositoires, solutions ou emplâtres, etc.
